# EUROPEAN PATENT SPECIFICATION

(11) **EP 0 717 616 B1**
(45) Date of publication and mention of the grant of the patent: **21.03.2001**
(21) Application number: 94926421.2
(22) Date of filing: 25.08.1994
(51) Int. Cl.: A61K 9/14, A61K 9/72, A61K 47/12, B01J 2/28

(54) **PROCESS FOR CONDITIONING SUBSTANCES**
VERFAHREN ZUR KONDITIONIERUNG VON SUBSTANZEN
PROCEDE POUR CONDITIONNER DES SUBSTANCES

(30) Priority: 27.08.1993 SE 9302777
(43) Date of publication of application: 26.06.1996
(73) Proprietor: AstraZeneca AB, 151 85 Södertälje (SE)
(72) Inventor: TROFAST, Eva, Ann-Christin, S-226 47 Lund (SE); BRIGGNER, Lars-Erik, S-226 54 Lund (SE)
(86) International application number: PCT/SE94/00780
(87) International publication number: WO 95/05805

(56) References cited:
- EP-A- 0 508 969
- WO-A-84/00294
- WO-A-91/16882

## Description

The present invention relates to a process for providing a fine-grained substance or substance mixture, which can be produced, stored and used while maintaining the aerodynamic properties required for inhalation of such a substance or substance mixture and which have improved physicochemical properties in the dry state, thereby facilitating the technical handling and significantly increase the medical value of the formulation used.

### Background of the invention

There are presently several effective drugs available for the treatment of patients with asthma or other respiratory disorders. It has been recognized that these drugs should be given by the inhaled route whenever possible. The ideal delivery system for inhalable drugs would be a user- and environment-friendly multidose inhaler giving accurate doses of a stable formulation with good aerodynamic behaviour of the particles.

During the past few years, there have been frequent demonstrations of the fact that the appropriate selection of the most suitable crystalline modification significantly can influence the clinical results of a given chemical substance. The chemical and physical stability of a solid in a particular dosage form can be improved by presenting the substance(s) in the appropriate crystal form. The solid state phase transformation of the substance in a dosage form can dramatically alter the pharmaceutical properties of the formulation. The solid state phase of the administered substance(s) can influence such important factors as bioavailability and physicochemical stability (specific surface area, particle size etc). Chemical stability in solid state and hygroscopicity are often closely related to the crystallinity.

Solid state transformations may occur during mechanical processing e.g. micronization. In a micronization process of solids, disruption or activation of the crystalline structure often leads to varying degrees of disorder through the formation of defects or amorphous regions. Such regions are often more sensitive to external effects e.g. moisture. It is necessary to establish the conditions whereby different forms of a substance might be converted to a single stable form thus eliminating differences in solid state properties and subsequent different physicochemical and pharmaceutical properties.

The increasing production and use of fine powders in the pharmaceutical industry has highlighted the need of reliable methods for assessing their physicochemical and technical handling. Mixing of cohesive powders will be influenced by the interparticulate forces between particles of the same species and also between particles of different species. Since fine powders agglomerate, the mixture will often be inhomogeneous, particularly the minor component will show a skewed distribution. One reason could be that the agglomerates of the minor component is not completely dispersed into their component particles; see further Chem. Eng. (1973), 12-19. Cohesive powders are thus very difficult to mix to a homogenous mixture in an accurate way, especially when one component is present only as a small fraction.

Substances will often be obtained in an amorphous state or a metastable crystalline form when spray drying, freeze drying, rapid solvent quenching or when using controlled precipitation, where both crystalline and amorphous forms can be prepared. The use of an amorphous form or metastable crystalline form is often limited due to its thermodynamic instability. It is therefore a desire to convert the amorphous form or the metastable crystalline form to the more stable crystalline state. For crystalline substances, a diminution operation step will give amorphous regions of the particle making the particle more sensitive to moisture and chemical degradation. The present invention deals with such physical changes, or more importantly, to anticipate them and the means by which these solid state phenomena can be handled.

The rearrangement or conditioning of a water-soluble substance, amorphous or partly amorphous, using a solvent like ethanol, acetone or the like has been described in Eur. Pat. Appl. EP 508 969 where single compounds have been applied. However, that method is not applicable for some substances containing crystal water, since organic solvents will eliminate the water thereby changing the properties of the substance considerably. It has been understood that water-soluble substances could not be conditioned by water while keeping the particle distribution of a fine-grained substance intact.

### References:

Amorphous-to-Crystalline Transformation of Sucrose, Phar. Res., 7(12), 1278 (1990) by J.T. Carstensen and K. Van Scoik.
Effect of Surface Characteristcs of Theophylline Anhydrate Powder on Hygroscopic Stability, J. Pharm. Pharmacol. 42, 606 (1990) by M. Otsuka et al.
Process for conditioning of water-soluble substances, Eur. Pat. Appl. 508969 by J. Trofast et al.
The molecular basis of moisture effect on the physical and chemical stability of drugs in the solid state, Int. J. Pharm. 62(1990), 87-95 by C. Ahlneck and G. Zografi.

### Brief description of the invention

The object of the invention is to provide a process for a fine-grained substance or substance mixture, which can be produced, stored and used while maintaining the aerodynamic properties required for inhalation of such a substance or substance mixture, whereby conditioning the mixture in a controlled process, thereby facilitating the technical handling and significantly increase the medical value of the formulation used.

### Detailed description of the invention

The object of the present invention is to provide a reliable process for providing a stable crystallinic form to a fine-grained substance or a substance mixture, which can be produced, stored and used while maintaining the aerodynamic properties required for inhalation of such a substance or a substance mixture. The process according to the present invention comprises the following steps:
a) in case of a substance mixture, preparing a homogenous mixture of the substances;
b) micronizing, direct precipitating or diminishing by any conventional method the substance or substance mixture into a particle size required for inhalation, the particle size being less than 10µm;
c) optionally preparing a homogenous mixture of the desired substances when each substance has been introduced from stage b) as separate fine-grained particles;
d) conditioning said substance or substance mixture by treatment with a water-containing vapour phase at a temperature/relative humidity combination suppressing the glass temperature of the substance or substance mixture below the process temperature;
e) drying; and
f) optionally preparing a homogenous mixture of the desired substances when each substance has been introduced from stage e) as separate fine-grained particles.

The conditioning step is carried out by treatment with a water containing vapour phase. Said water containing vapour phase is a water vapour phase with or without any organic solvent vapour present.

The glass temperature (T_{g}) is the temperature at which the mobility of an amorphous material undergoes changes from an immobile glassy state to mobile rubbery state (phase transition).

The conditioning is generally carried out at a temperature between 0 and 100°C, preferably between 10 and 50°C. Of practical reasons the conditioning is often performed at ambient temperature. The relative humidity (RH) at which the conditioning is carried out is chosen so that the phase transition occurs, mainly above 35% RH, preferably above 50% RH, and most preferably above 75% RH. The time used is considerably influenced by the batch size, relative humidity and
packing etc and may be from minutes to days.

The final formulation may also include different additives, e.g. a substance which enhances the absorption of a pharmacologically active drug in the lung. The enhancers used can be any of a number of compounds which act to enhance absorption through the layer of epithelial cell lining the alveoli of the lung and into the adjacent pulmonary vasculature. Among the substances with known absorption-enhancing properties are surfactants, such as alkali salts of fatty acids, sodium tauro-dihydrofusidate, lecithins, sodium glycocholate, sodium taurocholate, octylglucopyranoside and the like.

Other additives in the formulation may be carriers, diluents, antioxidants, buffer salts and the like, all of which will be treated according to the process of the present invention.

The accuracy and reproducibility of doses are often not sufficient when using very small doses in an inhalation device. Therefore very potent drugs may be diluted with a carrier in order to get an amount of powder sufficient to obtain a reliable and reproducible dose. Such a carrier may be carbohydrates like lactose, glucose, fructose, galactose, trehalose, sucrose, maltose, raffinose, maltitol, melezitose, starch, xylitol, mannitol, myoinositol, and the like and its hydrates, preferably lactose and mannitol, and amino acids such as alanine, betaine and the like.

Coarser particles having a size above 10 µm may also be conditioned using the process according to the present invention.

The present invention may be applied to for example the following pharmacologically active substances:

Formoterol (e.g. as fumarate) and salmeterol (e.g. as xinafoate) are highly selective long-acting β₂-adrenergic agonists having bronchospasmolytic effect and are effective in the treatment of reversible obstructive lung ailments of various genesis, particularly asthmatic conditions. Salbutamol (e.g. as sulphate), bambuterol (e.g. as hydrochloride), terbutaline (e.g. as sulphate), fenoterol (e.g. as hydrobromide), clenbuterol (e.g. as hydrochloride), procaterol (e.g. as hydrochloride), bitolterol (e.g. as mesylate) and broxaterol are highly selective β₂-adrenergic agonists and ipratropium bromide is an anticholinergic bronchodilator. Examples on antiinflammatory glucocorticoids are budesonide, (22R)-6α,9α-difluoro-11β,21-dihydroxy-16α,17α-propylmethylenedioxy-4-pregnen-3,20-dione, fluticasone (e.g. as propionate ester), beclomethasone (e.g. as dipropionate ester), tipredane, momethasone and the like. Several of the compounds could be in the form of pharmacologically acceptable esters, salts, solvates, such as hydrates, or solvates of such esters or salts, if any.

The preferred substances to which the invention is to be applied are terbutaline sulphate, salbutamol sulphate, fenoterol hydrobromide, ipratropium bromide, bambuterol hydrochloride, formoterol fumarate and salmeterol xinafoate, and their solvates, especially their hydrates.

The most preferred substance mixture to which the invention is to be applied is formoterol (as formoterol fumarate dihydrate)/lactose (monohydrate), although the same principle may be applied to combinations such as salbutamol (as salbutamol sulphate)/lactose, terbutaline (as terbutaline sulphate)/lactose, ipratropium bromide/lactose, budesonide/lactose, (22R)-6α,9α-difluoro-11β,21-dihydroxy-16α,17α-propylmethylenedioxy-4-pregnen-3,20-dione/mannitol, (22R)-6α,9α-difluoro-11β,21-dihydroxy-16α,17α-propylmethylenedioxy-4-pregnen-3,20-dione/myoinositol and (22R) -6α,9α-difluoro-11β,21-dihydroxy-16α,17α-propylmethylenedioxy-4-pregnen-3,20-dione/lactose. When one of the components is rather insoluble in water, it is possible to use an organic solvent as a conditioning agent for one compound and water vapour as a conditioning agent for the other one in the conditioning step. In that case the conditioning may be carried out in a two step procedure wherein the first step is conditioning with an organic solvent followed by conditioning by water vapour in a second step; or vice versa.

The rearrangement or conditioning of the substance or substance mixture, amorphous or partly amorphous, involve treatment of the substance(s) with a water containing vapour phase in a controlled fashion. This conditioning step is to be performed in a defined environment with controlled and adjustable humidity or a column using inert gas and/or organic solvent vapour containing the required amount of water vapour. The packing of the substance or substance mixture affects the time needed as well as the result of the conditioning. The tendency of caking is affecting the number and size of particles. In case of a substance mixture, it is usually an advantage to mix the substances before the micronizing step in order to ensure a homogenous mixture when using small ratios between the drug substance and the additive.

With the present invention it is possible to condition two or more substances in the same process while the particle distribution is maintained and this is from a technical standpoint a great advantage.

The ratio between the substances in a substance mixture is between 1:1 and 1:1000, preferably between 1:1 and 1:500, and most preferred between 1:1 and 1:200 in the case where one substance is a pharmacologically active substance and the other one is an additive.

The particle size of the fine-grained substances should be identical before and after the conditioning step as measured by different instruments like Malvern Master Sizer, Coulter Counter or a microscope.

It is also of utmost importance that the particles obtained are well-defined in size and distribution as well as have small batch to batch variations in order to obtain agglomerates that will completely disintegrate into its primary particles in the inhaler used.

It is an object of the present invention to provide a reliable process, where the drug formulation of a single drug substance or a combination of a drug substance/additive, preferably formoterol fumarate dihydrate/lactose can be conveniently and reproducibly prepared.

It is a further object of the present invention to provide pharmaceutical formulations comprising formoterol fumarate dihydrate, salbutamol sulphate, or ipratropium bromide having a particle size of less than 10 µm and an additive, which when subjected to water-containing vapour gives off heat of less than 0.5 J/g. The additive may be lactose or melezitose, and the particle size of the additive may also be less than 10 µm.

It is yet another object of the present invention to provide formoterol fumarate dihydrate, lactose and melezitose having a particle size less than 10 µm, which when subjected to water-containing vapour gives off heat of less than 0.5 J/g.

For some material such as formoterol/lactose, where the T_{g} (the glass transition temperature, the temperature at which the mobility of an amorphous substance undergoes changes from an immobile glassy state to mobile rubbery state) or water sensitivity is markedly different for the drug substance and the additive, the process can be performed in two subsequent steps, i.e.
conditioning of one substance at one temperature/RH combination followed by conditioning at a higher temperature/RH for a second substance.

The mixing step is preferably performed before the micronization step in order to ensure the content uniformity or in a single step using a vibratory ball mill as reported by I. Krycer and J.A. Hersey in Int. J. Pharm. 6, 119-129 (1980). It is also possible to mix the substances after micronization or after each substance has been conditioned.

In some instances it has been possible to use infrared spectroscopy in order to study the conversion of an amorphous form or a partly crystalline form into a stable crystalline form. Other methods available include BET gas adsorption, X-ray powder diffraction, isothermal microcalorimetry and differential scanning calorimetry (DSC). We have found that BET gas adsorption and isothermal microcalorimetry being the best methods for distinguishing the different forms of the tested compounds.

When a substance or substance mixture is agglomerated and used as such, a drop of about 70-80% of the respirable particles is found when exposed to high humidity. It has astonishly been found that a drop of only about 25-30% occurs when a substance or substance mixture has been conditioned (at 50% RH for formoterol fumarate dihydrate/lactose mixture) before agglomeration and exposed to high humidity. After further conditioning at 75% RH a drop of only 5-10% of the respirable particles will occur. There is no difference in particle distribution as measured by a Malver instrument before and after conditioning at 75% RH. If the conditioning is performed with the agglomerated product the particle distribution is considerable worse and the formulation useless in an inhalation device.

### Experimental procedure

The invention relates to the following procedure:
1. Mixing the drug substance with the additive in a defined ratio.
2. Micronizing the mixture.
3. Conditioning at a temperature/relative humidity combination, which suppresses the glass temperature of substances involved below the process temperature. The glass temperature (T_{g}) is the temperature at which the mobility of an amorphous material undergoes changes from an immobile glassy state to mobile rubbery state.
4. Drying with dry nitrogen or air, or in vacuum.

### EXAMPLES

The invention is further illustrated but not limited by the following examples performed according to the described experimental procedure. Several batches of each substance or substance mixture have been measured. The data represents a comparison of the heat (J/g) given off by non-conditioned and conditioned substances when subjected to a water containing vapour phase. The experiments are performed by using a Thermal Activity Monitor 2277 (Thermometrics AB, Sweden).

### Example 1

| | |
|---|---|
| Salbutamol sulphate (25%)/lactose (75%) | |
| Conditioned at relative humidity (RH) | 50-60 % RH |
| Non-conditioned substance (J/g) | 5-8 |
| Conditioned substance (J/g) | <0.5 |

### Example 2

| | |
|---|---|
| Ipratropium bromide (6%)/lactose (94%) | |
| Conditioned at relative humidity (RH) | 50-60 % RH |
| Non-conditioned substance (J/g) | 6-8 |
| Conditioned substance (J/g) | <0.5 |

### Example 3

| | |
|---|---|
| Formoterol fumarate dihvdrate | |
| Conditioned at relative humidity (RH) | 75 % RH |
| Non-conditioned substance (J/g) | 6 |
| Conditioned substance (J/g) | <0.5 |

### Example 4

| | |
|---|---|
| Lactose (see Figure 1) | |
| Conditioned at relative humidity (RH) | 50 % RH |
| Non-conditioned substance (J/g) | 10-14 |
| Conditioned substance (J/g) | <0.5 |

### Example 5

| | |
|---|---|
| Melezitose | |
| Conditioned at relative humidity (RH) | 50 % RH |
| Non-conditioned substance (J/g) | 12 |
| Conditioned substance (J/g) | <0.5 |

### Example 6

| | |
|---|---|
| Formoterol fumarate dihydrate (2%)/lactose (98%) | |
| Conditioned at relative humidity (RH) | 50 % RH |
| Non-conditioned substance (J/g) | 10-14 |
| Conditioned substance (J/g) | <0.5 |

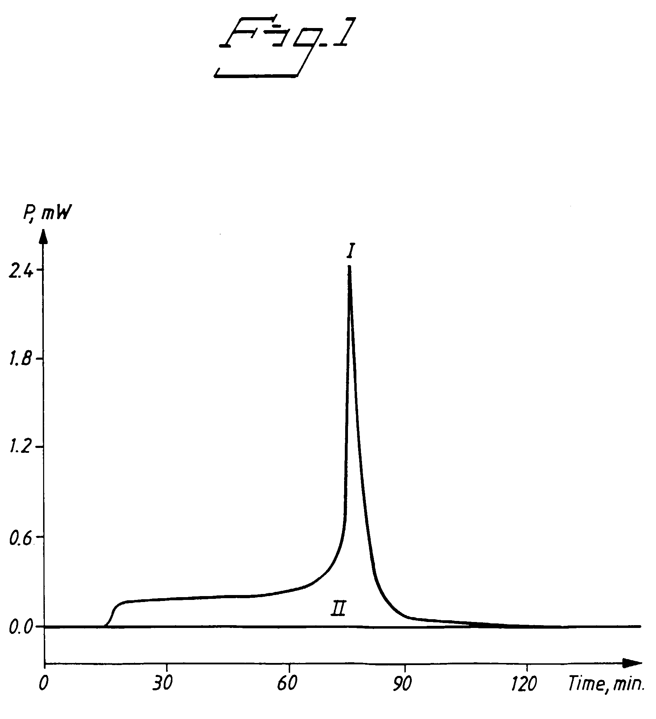

During a recrystallization a large amount of heat is evolved, and by monitoring the calometrical signal the sample is checked for any amorphous content. Figure 1 shows micronised lactose before (I) and after (II) conditioning. Thus, a complete crystallinity has been obtained during the conditioning according to the invention.

## Claims

1. A process for providing a stable crystalline form of a fine-grained substance or a substance mixture, which can be produced, stored and used while maintaining the aerodynamic properties required for inhalation of such a substance or a substance mixture, **characterized** in
a) in case of a substance mixture, preparing a homogenous mixture of the substances;
b) micronizing, direct precipitating or diminishing by any conventional method the substance or substance mixture into a particle size required for inhalation, the particle size being less than 10 µm;
c) optionally preparing a homogenous mixture of the desired substances when each substance has been introduced from stage b) as separate fine-grained particles;
d) conditioning said substance or substance mixture by treatment with a water-containing vapour phase at a temperature/relative humidity combination suppressing the glass temperature of the substance or substance mixture below the process temperature;
e) drying; and
f) optionally preparing a homogenous mixture of the desired substances when each substance has been introduced from stage e) as separate fine-grained particles.

2. A process according to claim 1, wherein the conditioning, in the case of a substance mixture, is performed in a one step procedure, or in a multistep procedure using different relative humidity/temperature combinations.

3. A process according to any one of the preceding claims, wherein the substance is a single drug substance or a combination of a drug substance and an additive.

4. A process according to any one of the preceding claims, wherein the substance is selected from the group consisting of formoterol, salmeterol, salbutamol, bambuterol, terbutaline, fenoterol, clenbuterol, procaterol, bitolterol, broxaterol, ipratropium bromide, budesonide, (22R)-6α,9α-difluoro-11β,21-dihydroxy-16α,17α-propylmethylenedioxy-4-pregnen-3,20-dione, fluticasone, beclomethasone, tipredane, momethasone, and pharmacologically acceptable esters, salts and solvates thereof and solvates of such esters or salts.

5. A process according to claim 4, wherein the substance is selected from the group consisting of formoterol fumarate, salmeterol xinafoate, salbutamol sulphate, bambuterol hydrochloride, terbutaline sulphate, fenoterol hydrobromide, clenbuterol hydrochloride, procaterol hydrochloride, bitolterol mesylate, fluticasone propionate, beclomethasone dipropionate and solvates of any one thereof.

6. A process according to claim 3, wherein the additive is selected from the group consisting of lactose, glucose, fructose, galactose, trehalose, sucrose, maltose, raffinose, maltitol, melezitose, starch, xylitol, mannitol, myoinositol and hydrates of any one thereof, and amino acids, such as alanine and betaine.

7. A process according to claim 3, wherein the additive is selected from the group consisting of lactose, and mannitol, and a hydrate of either thereof.

8. A process according to claim 3, wherein the additive is selected from the group consisting of enhancers, antioxidants and buffer salts.

9. A process according to claim 8, wherein the additive is an enhancer selected from the group consisting of alkali salts of fatty acids, sodium taurodihydrofusidate, lecithins, sodium glycocholate, sodium taurocholate and octylglucopyranoside.

10. A process according to any one of claims 1 to 3, wherein the substance is a substance mixture selected from formoterol/lactose, salbutamol/lactose, terbutaline/lactose, ipratropium bromide/lactose, budesonide/lactose, (22R)-6α,9α-difluoro-11β,21-dihydroxy-16α,17α-propylmethylene-dioxy-4-pregnen-3,20-dione/mannitol,(22R)-6α,9α-difluoro-11β,21-dihydroxy-16α,17α-propylmethylenedioxy-4-pregnen-3,20-dione/myoinositol and (22R) -6α,9α-difluoro-11β,21-dihydroxy-16α,17α-propyl-methylenedioxy-4-pregnen-3,20-dione/lactose.

11. A process according to any one of claims 1 to 3, wherein the substance is a substance mixture selected from formoterol fumarate dihydrate/lactose, salbutamol sulphate/lactose and terbutaline sulphate/lactose.

12. A process according to any one of the preceding claims, wherein step d) is carried out at a temperature between 0 and 100°C, preferably between 10 and 50°C and at a relative humidity so as that the phase transition occurs, mainly above 35% RH, preferably above 50% RH, and most preferably above 75% RH.

13. A process according to claim 1, wherein the ratio between the substances in a substance mixture is between 1:1 and 1:1000, preferably between 1:1 and 1:500, and most preferred between 1:1 and 1:200 in the case where one substance is a pharmacologically active substance and the other one is an additive.

14. Formoterol fumarate dihydrate having a particle size less than 10 µm, which when subjected to water-containing vapour gives off heat of less than 0.5 J/g.

15. Lactose having a particle size less than 10 µm, which when subjected to water-containing vapour gives off heat of less than 0.5 J/g.

16. Melezitose having a particle size less than 10 µm, which when subjected to water-containing vapour gives off heat of less than 0.5 J/g.

17. A pharmaceutical formulation comprising formoterol fumarate dihydrate having a particle size of less than 10 µm and an additive, which when subjected to water-containing vapour gives off heat of less than 0.5 J/g.

18. A pharmaceutical formulation comprising salbutamol sulphate having a particle size of less than 10 µm and an additive, which when subjected to water-containing vapour gives off heat of less than 0.5 J/g.

19. A pharmaceutical formulation comprising ipratropium bromide having a particle size of less than 10 µm and an additive, which when subjected to water-containing vapour gives off heat of less than 0.5 J/g.

20. The formulation according to any of claims 17 to 19, wherein the additive has a particle size of less than 10 µm.

21. The formulation according to any of claims 17 to 20, wherein the additive is lactose.

22. The formulation according to any of claims 17 to 21, wherein the additive is melezitose.

## Patentansprüche

1. Verfahren zur Bereitstellung einer stabilen kristallinen Form einer feinkörnigen Substanz oder Substanzmischung, die unter Erhaltung der für die Inhalation einer derartigen Substanz oder Substanzmischung erforderlichen aerodynamischen Eigenschaften hergestellt, gelagert und verwendet werden kann, dadurch gekennzeichnet, daß man
a) im Fall einer Substanzmischung eine homogene Mischung der Substanzen herstellt;
b) die Substanz oder Substanzmischung nach einem herkömmlichen Verfahren auf eine für die Inhalation erforderliche Teilchengröße mikronisiert, direkt ausfällt oder zerkleinert, wobei die Teilchengröße weniger als 10 µm beträgt;
c) gegebenenfalls eine homogene Mischung der gewünschten Substanzen herstellt, wenn jede Substanz aus Stufe b) in Form von separaten feinkörnigen Teilchen eingetragen worden ist;
d) die Substanz oder Substanzmischung durch Behandlung mit einer wasserhaltigen Dampfphase bei einer Kombination von Temperatur und relativer Feuchte, die die Glastemperatur der Substanz oder Substanzmischung unter die Verfahrenstemperatur senkt, konditioniert;
e) trocknet und
f) gegebenenfalls eine homogene Mischung der gewünschten Substanzen herstellt, wenn jede Substanz aus Stufe e) in Form von separaten feinkörnigen Teilchen eingetragen worden ist.

2. Verfahren nach Anspruch 1, bei dem man die Konditionierung im Fall einer Substanzmischung in einem Ein- oder Mehrstufenverfahren unter Verwendung verschiedener Kombinationen von relativer Feuchte und Temperatur durchführt.

3. Verfahren nach einem der vorhergehenden Ansprüche, bei dem man als Substanz einen einzigen Arzneistoff oder eine Kombination aus einem Arzneistoff und einem Additiv einsetzt.

4. Verfahren nach einem der vorhergehenden Ansprüche, bei dem man die Substanz aus der Gruppe bestehend aus Formoterol, Salmeterol, Salbutamol, Bambuterol, Terbutalin, Fenoterol, Clenbuterol, Procaterol, Bitolterol, Broxaterol, Ipratropiumbromid, Budesonid, (22R)-6α,9α-Difluor-11β,21-dihydroxy-16α,17α-propylmethylendioxy-4-pregnen-3,20-dion, Fluticason, Beclomethason, Tipredan, Momethason und pharmakologisch unbedenklichen Estern, Salzen und Solvaten davon sowie Solvaten derartiger Ester oder Salze auswählt.

5. Verfahren nach Anspruch 4, bei dem man die Substanz aus der Gruppe bestehend aus Formoterolfumarat, Salmeterolxinafoat, Salbutamolsulfat, Bambuterolhydrochlorid, Terbutalinsulfat, Fenoterolhydrobromid, Clenbuterolhydrochlorid, Procaterolhydrochlorid, Bitolterolmesylat, Fluticasonpropionat, Beclomethasondipropionat und Solvaten davon auswählt.

6. Verfahren nach Anspruch 3, bei dem man das Additiv aus der Gruppe bestehend aus Lactose, Glucose, Fructose, Galactose, Trehalose, Saccharose, Maltose, Raffinose, Maltit, Melezitose, Stärke, Xylit, Mannit, Myoinosit und Hydraten davon sowie Aminosäuren, wie Alanin und Betain, auswählt.

7. Verfahren nach Anspruch 3, bei dem man das Additiv aus der Gruppe bestehend aus Lactose und Mannit sowie Hydraten davon auswählt.

8. Verfahren nach Anspruch 3, bei dem man das Additiv aus der Gruppe bestehend aus Resorptionsverbesserern, Antioxidantien und Puffersalzen auswählt.

9. Verfahren nach Anspruch 8, bei dem man als Additiv einen Resorptionsverbesserer aus der Gruppe bestehend aus Fettsäurealkalisalzen, Natriumtaurodihydrofusidat, Lecithinen, Natriumglycocholat, Natriumtaurocholat und Octylglucopyranosid einsetzt.

10. Verfahren nach einem der Ansprüche 1 bis 3, bei dem man als Substanz eine Substanzmischung aus der Gruppe Formoterol/Lactose, Salbutamol/Lactose, Terbutalin/Lactose, Ipratropiumbromid/Lactose, Budesonid/Lactose, (22R)-6α,9α-Difluor-11β,21dihydroxy-16α,17α-propylmethylendioxy-4-pregnen-3,20dion/Mannit, (22R)-6α,9α-Difluor-11β,21-dihydroxy16α,17α-propylmethylendioxy-4-pregnen-3,20dion/Myoinosit und (22R)-6α,9α-Difluor-11β,21dihydroxy-16α,17α-propylmethylendioxy-4-pregnen-3,20dion/Lactose einsetzt.

11. Verfahren nach einem der Ansprüche 1 bis 3, bei dem man als Substanz eine Substanzmischung aus der Gruppe Formoterolfumaratdihydrat/Lactose, Salbutamolsulfat/Lactose und Terbutalinsulfat/Lactose einsetzt.

12. Verfahren nach einem der vorhergehenden Ansprüche, bei dem man Schritt d) bei einer Temperatur zwischen 0 und 100°C, vorzugsweise zwischen 10 und 50°C, und bei einer solchen relativen Feuchte durchführt, daß der Phasenübergang stattfindet, hauptsächlich bei über 35% RF, vorzugsweise bei über 50% RF und ganz besonders bevorzugt bei über 75% RF.

13. Verfahren nach Anspruch 1, bei dem das Verhältnis zwischen den Substanzen in einer Substanzmischung zwischen 1:1 und 1:1000, vorzugsweise zwischen 1:1 und 1:500 und ganz besonders bevorzugt zwischen 1:1 und 1:200 liegt, wenn es sich bei einer Substanz um einen pharmakologischen Wirkstoff und bei der anderen um ein Additiv handelt.

14. Formoterolfumaratdihydrat mit einer Teilchengröße von weniger als 10 µm, das bei Einwirkung von wasserhaltigem Dampf weniger als 0,5 J/g Wärme abgibt.

15. Lactose mit einer Teilchengröße von weniger als 10 µm, die bei Einwirkung von wasserhaltigem Dampf weniger als 0,5 J/g Wärme abgibt.

16. Melezitose mit einer Teilchengröße von weniger als 10 µm, die bei Einwirkung von wasserhaltigem Dampf weniger als 0,5 J/g Wärme abgibt.

17. Pharmazeutische Formulierung, enthaltend Formoterolfumaratdihydrat mit einer Teilchengröße von weniger als 10 µm und ein Additiv, das bei Einwirkung von wasserhaltigem Dampf weniger als 0,5 J/g Wärme abgibt.

18. Pharmazeutische Formulierung, enthaltend Salbutamolsulfat mit einer Teilchengröße von weniger als 10 µm und ein Additiv, das bei Einwirkung von wasserhaltigem Dampf weniger als 0,5 J/g Wärme abgibt.

19. Pharmazeutische Formulierung, enthaltend Ipratropiumbromid mit einer Teilchengröße von weniger als 10 µm und ein Additiv, das bei Einwirkung von wasserhaltigem Dampf weniger als 0,5 J/g Wärme abgibt.

20. Formulierung nach einem der Ansprüche 17 bis 19, in der das Additiv eine Teilchengröße von weniger als 10 µm aufweist.

21. Formulierung nach einem der Ansprüche 17 bis 20, in der es sich bei dem Additiv um Lactose handelt.

22. Formulierung nach einem der Ansprüche 17 bis 21, in der es sich bei dem Additiv um Melezitose handelt.

## Revendications

1. Procédé pour obtenir une forme cristalline stable d'une substance à grains fins ou d'un mélange de substances, qui peut être produite, stockée et utilisée tout en conservant les propriétés aérodynamiques nécessaires à l'inhalation d'une telle substance ou d'un tel mélange de substances, **caractérisé** par
a) dans le cas d'un mélange de substances, la préparation d'un mélange homogène des substances;
b) la micronisation, la précipitation directe ou la fragmentation, par tout procédé classique, de la substance ou du mélange de substances, en une granulométrie nécessaire à l'inhalation, la granulométrie étant inférieure à 10 µm;
c) la préparation éventuelle d'un mélange homogène des substances souhaitées lorsque chaque substance a été introduite à partir de l'étape b) sous forme de particules à grains fins séparées;
d) le conditionnement de ladite substance ou dudit mélange de substances par traitement avec une phase vapeur contenant de l'eau à une combinaison température/humidité relative abaissant la température de transition vitreuse de la substance ou du mélange de substances au-dessous de la température du procédé;
e) le séchage; et
f) éventuellement la préparation d'un mélange homogène des substances souhaitées lorsque chaque substance a été introduite à partir de l'étape e) sous forme de particules à grains fins séparées.

2. Procédé selon la revendication 1, dans lequel le conditionnement, dans le cas d'un mélange de substances, est réalisé dans un procédé à une étape, ou dans un procédé à plusieurs étapes, avec différentes combinaisons humidité relative/température.

3. Procédé selon l'une quelconque des revendications précédentes, dans lequel la substance est une seule substance médicamenteuse ou une combinaison d'une substance médicamenteuse et d'un additif.

4. Procédé selon l'une quelconque des revendications précédentes, dans lequel la substance est choisie dans le groupe constitué par le formotérol, le salmétérol, le salbutamol, le bambutérol, la terbutaline, le fénotérol, le clenbutérol, le procatérol, le bitoltérol, le broxatérol, le bromure d'ipratropium, le budésonide, la (22R)-6α,9α-difluoro11β,21-dihydroxy-16α,17α-propylméthylènedioxy-4prégnène-3,20-dione, la fluticasone, la béclométhasone, le tiprédane, la mométhasone et leurs esters, sels et solvates pharmacologiquement acceptables, et les solvates de ces esters ou sels.

5. Procédé selon la revendication 4, dans lequel la substance est choisie dans le groupe constitué par le fumarate de formotérol, le xinafoate de salmétérol, le sulfate de salbutamol, le chlorhydrate de bambutérol, le sulfate de terbutaline, le bromhydrate de fénotérol, le chlorhydrate de clenbutérol, le chlorhydrate de procatérol, le mésylate de bitoltérol, le propionate de fluticasone, le dipropionate de béclométhasone et des solvates de l'un quelconque de ceux-ci.

6. Procédé selon la revendication 3, dans lequel l'additif est choisi dans le groupe constitué par le lactose, le glucose, le fructose, le galactose, le tréhalose, le saccharose, le maltose, le raffinose, le maltitol, le mélézitose, l'amidon, le xylitol, le mannitol, le myoinositol et des hydrates quelconques de ceux-ci, et des acides aminés, tels que l'alanine et la bétaïne.

7. Procédé selon la revendication 3, dans lequel l'additif est choisi dans le groupe constitué par le lactose et le mannitol, et un hydrate quelconque de ceux-ci.

8. Procédé selon la revendication 3, dans lequel l'additif est choisi dans le groupe constitué par les exhausteurs, les antioxydants et les sels tampons.

9. Procédé selon la revendication 8, dans lequel l'additif est un exhausteur choisi dans le groupe constitué par les sels alcalins d'acides gras, le taurodihydrofusidate de sodium, les lécithines, le glycocholate de sodium, le taurocholate de sodium et l'octylglucopyranoside.

10. Procédé selon l'une quelconque des revendications 1 à 3, dans lequel la substance est un mélange de substances choisi parmi formotérol/lactose, salbutamol/lactose, terbutaline/lactose, bromure d'ipratropium/lactose, budésonide/lactose, (22R)-6α,9αdifluoro-11β,21-dihydroxy-16α,17α-propylméthylènedioxy-4-prégnène-3,20-dione/mannitol, (22R)-6α,9αdifluoro-11β,21-dihydroxy-16α,17α-propylméthylènedioxy-4-prégnène-3,20-dione/myoinositol et (22R)-6α,9α-difluoro-11β,21-dihydroxy-16α,17α-propylméthylènedioxy-4-prégnène-3,20-dione/lactose.

11. Procédé selon l'une quelconque des revendications 1 à 3, dans lequel la substance est un mélange de substances choisi parmi fumarate de formotérol dihydraté/lactose, sulfate de salbutamol/ lactose et sulfate de terbutaline/lactose.

12. Procédé selon l'une quelconque des revendications précédentes, dans lequel l'étape d) est réalisée à une température comprise entre 0 et 100°C, de préférence entre 10 et 50°C, et à une humidité relative telle que la transition de phase ait lieu, principalement supérieure à 35% de HR, de préférence supérieure à 50% de HR et tout particulièrement supérieure à 75% de HR.

13. Procédé selon la revendication 1, dans lequel le rapport entre les substances dans un mélange de substances est compris entre 1:1 et 1:1 000, de préférence entre 1:1 et 1:500 et tout particulièrement entre 1:1 et 1:200 dans le cas où une substance est une substance ayant une activité pharmacologique et l'autre est un additif.

14. Fumarate de formotérol dihydraté ayant une granulométrie inférieure à 10 µm, qui dégage, lorsqu'il est soumis à de la vapeur contenant de l'eau, moins de 0,5 J/g de chaleur.

15. Lactose ayant une granulométrie inférieure à 10 µm, qui dégage, lorsqu'il est soumis à de la vapeur contenant de l'eau, moins de 0,5 J/g de chaleur.

16. Mélézitose ayant une granulométrie inférieure à 10 µm, qui dégage, lorsqu'il est soumis à de la vapeur contenant de l'eau, moins de 0,5 J/g de chaleur.

17. Formulation pharmaceutique comprenant du fumarate de formotérol dihydraté ayant une granulométrie inférieure à 10 µm et un additif, qui dégage, lorsqu'elle est soumise à de la vapeur contenant de l'eau, moins de 0,5 J/g de chaleur.

18. Formulation pharmaceutique comprenant du sulfate de salbutamol ayant une granulométrie inférieure à 10 µm et un additif, qui dégage, lorsqu'elle est soumise à de la vapeur contenant de l'eau, moins de 0,5 J/g de chaleur.

19. Formulation pharmaceutique comprenant du bromure d'ipratropium ayant une granulométrie inférieure à 10 µm et un additif, qui dégage, lorsqu'elle est soumise à de la vapeur contenant de l'eau, moins de 0,5 J/g de chaleur.

20. Formulation selon l'une quelconque des revendications 17 à 19, dans laquelle l'additif possède une granulométrie inférieure à 10 µm.

21. Formulation selon l'une quelconque des revendications 17 à 20, dans laquelle l'additif est le lactose.

22. Formulation selon l'une quelconque des revendications 17 à 21, dans laquelle l'additif est le mélézitose.
